## Europäisches Patentamt

## European Patent Office

## Office européen des brevets

(19)

(11) Publication number: **0 532 272 A2**

# EUROPEAN PATENT APPLICATION

(12)

(21) Application number: **92308133.5**

(22) Date of filing: **08.09.92**

(51) Int. Cl.⁵: **A61K 7/08, A61K 7/06**

(30) Priority: **12.09.91 GB 9119516**

(43) Date of publication of application:
**17.03.93 Bulletin 93/11**

(84) Designated Contracting States:
**AT BE CH DE DK ES FR GB GR IE IT LI NL PT SE**

(71) Applicant: **UNILEVER PLC**
**Unilever House Blackfriars**
**London EC4P 4BQ (GB)**
(84) **GB IE**

(71) Applicant: **UNILEVER N.V.**
**Weena 455**
**NL-3013 AL Rotterdam (NL)**
(84) **BE CH DE DK ES FR GR IT LI NL PT SE AT**

(72) Inventor: **Tan-Walker, Ruby Loo Bick**
**16 School Lane, Guilden**
**Sutton, Chester CH3 7ET (GB)**

(74) Representative: **Ford, Michael Frederick et al**
**MEWBURN ELLIS 2 Cursitor Street**
**London EC4A 1BQ (GB)**

(54) **Hair conditioning composition.**

(57) A transparent hair conditioning composition comprises:
(a) a cationic surfactant component and
(b) a highly alkoxylated anionic surfactant component.
The highly alkoxylated anionic surfactant components contains at least about 5 EO/PO units per molecule.

EP 0 532 272 A2

The present invention relates to hair conditioning compositions, more particularly to hair conditioning compositions which are transparent.

Hair conditioning compositions may be formulated to select those components which together result in compositions which possess a desirable combination of properties. In particular, as well as exhibiting good conditioning properties, e.g. imparting smoothness and/or softness to the hair, ease of wet and/or dry combing thereof and anti-static properties, hair conditioning compositions are often desired to be transparent, i.e. optically clear or substantially clear.

The latter property is not only of benefit as regards visual product appeal to the consumer, but also gives a less coated feel to the hair, which is particularly important for people whose hair is naturally greasy and for whom conventional opaque conditioners are often undesirable because of heavy coating and excessive conditioning perceptions.

One particular known hair conditioning composition which is transparent is disclosed in JP-A-52-122638 and comprises a cationic surfactant component, particularly cetyltrimethylammonium chloride, and a phosphoric acid ester component, particularly a neutralised mono/diphosphate ester of C18 (3EO) alcohol.

In the disclosed compositions, the preferred weight ratio of cationic surfactant component to phosphoric acid ester component is between 1:1 and 4:1. Based on this teaching, an optimum weight ratio of cationic surfactant component to phosphoric acid ester component has been found to be approximately 1.5:1, corresponding to a molar ratio of approximately 3:1. However, such compositions have been recognised as having limited hair conditioning properties.

It has also been found that it is not possible to simply increase the proportion of phosphoric acid ester component with the aim of enhancing the hair conditioning benefits imparted by the composition, since that results in a cloudy product, which, as discussed above, is undesirable.

EP-A-0100164 discloses hair conditioning preparations comprising anionic surfactant and a cationic polymer, rendered clear by inclusion of a clarifying agent. When diluted with water upon use, an insoluble complex is formed between the cationic polymer and anionic surfactant, which is precipitated and thereby deposited onto the hair.

US 4744977 discloses hair conditioning compositions comprising particular $\beta$-hydroxy quaternary ammonium compounds. These hair conditioning compounds are indicated as being more compatible with high-foam anionic surfactants compared with other known quaternary ammonium compounds, giving products which are less cloudy and less prone to precipitation of insoluble deposits. The reference discloses generally a wide variety of anionic surfactants for use in combination with the defined cationic surfactant in hair conditioning shampoos.

Surprisingly, it has now been found that hair conditioning compositions, particularly shampoos, which comprise a wide variety of cationic surfactant components and which are optically clear or translucent without the need for inclusion of clarifying agents or transparency materials, can be prepared by incorporation also of an anionic surfactant component which is highly alkoxylated.

Accordingly, in one aspect the present invention provides a hair conditioning composition comprising:

(a) at least one cationic surfactant component, and

(b) at least one highly alkoxylated anionic surfactant component.

The present invention will now be described in detail.

As used herein, the term "highly alkoxylated" means having at least about 5 alkylene oxide residues per molecule.

Preferably, the molar ratio of component (b) to component (a) is at least about 0.5:1, more preferably at least about 1:1. Even more preferably, this molar ratio is at least about 2:1 and may even be as high as about 5:1 or more.

Preferred hair conditioning compositions according to the invention are aqueous compositions containing, with or without other ingredients, from about 0.1 to about 90% by weight of the combined cationic and highly alkoxylated anionic surfactant components. Suitably, the compositions may contain from about 0.5 to about 20% by weight of the combined cationic and anionic surfactant components.

(a) Cationic surfactant component

Preferred cationic surfactants for use in the invention are quaternary ammonium hydroxides or salts thereof, e.g. chlorides. Examples of suitable cationic surfactants for use in the invention include: tetramethylammonium chloride, tetraethylammonium chloride, cetyltrimethylammonium chloride, cetylpyridinium chloride, octyltrimethylammonium chloride, dodecyltrimethylammonium chloride, hexadecyltrimethylammonium chloride, octyldimethylbenzylammonium chloride, decyldimethylbenzylammonium chloride, stearyldimethylbenzylammonium chloride, didodecyldimethylammonium chloride, dioctadecyldimethylammonium chloride, tallow trimethylammonium chloride, cocotrimethylammonium chloride, and the corresponding hydroxides thereof. Further suitable cationic surfactants include those materials identified by the CTFA definitions Quaternium-5, Quaternium-31 and Quaternium-18. Mixtures of any of the foregoing materials may also be suitable. A particularly preferred cationic surfactant for use in the invention is cetyltrimethylammonium chloride, which is available commercially for example as ARQUAD 16/50.

(b) Highly alkoxylated anionic surfactant component

Suitable highly alkoxylated anionic surfactants for use in the invention include the alkyl ether sulphates, alkyl ether phosphates and alkyl ether carboxylates which contain at least about 5 or 6, preferably at least about 7 or 8 alkylene oxide, preferably ethylene oxide, residues per molecule. Suitably, the anionic surfactants may contain from about 5 to 20 or more (e.g. up to about 30) ethylene oxide and/or (though less preferably) propylene oxide units per molecule. A preferred alkoxy residue content is from about 5 or 6 to about 10 EO/PO units per molecule.

Suitable highly alkoxylated alkyl ether sulphates are widely known in the art and widely available commercially. Examples include sodium or ammonium lauryl ether sulphate containing from 5 to 20 EO units per molecule.

Suitable highly alkoxylated phosphate esters are available commercially, for example as CRODAFOS N10A (mono/di-oleyl ethoxy phosphate, 10EO) CRODAFOS CS10 (mono-di-cetostearyl ethoxy phosphate, 10EO) and CRODAFOS G26A (polyol ether phosphate ester (mixture of mono/di esters), 26EO).

Suitable highly alkoxylated ether carboxylates are commercially available, for example as MARLINAT CM100 (C12-C14 fatty alcohol polyglycol ether carboxylic acid, 10EO).

(c) Other components

The hair conditioning compositions of the present invention may contain additional components usually found in conventional hair care compositions.

For instance, the hair conditioning compositions of the invention may contain a suitable amount of a thickening agent such as a polymeric thickener, such as hydroxyethylcellulose (available commercially as NATROSOL 250HHR).

The compositions of the invention may also optionally contain one or more additional surfactants selected from anionic, nonionic, amphoteric and zwitterionic surfactants, and mixtures thereof, and/or one or more additional conditioning agents.

Other optional components which may be present in the hair conditioning compositions of the invention include deposition aids, perfumes, colouring agents, anti-bacterial agents, anti-dandruff agents, preservatives, proteins, polymers, sunscreens, buffering agents, moisturisers and natural ingredients such as herb extracts.

The compositions of the present invention may be prepared by conventional mixing techniques, as are well known in the art.

The present invention is further illustrated by the following Examples.

EXAMPLES

A number of hair conditioning composition were prepared by conventional methods according to the formulations shown in the Table below. All amounts are in % by weight, unless otherwise stated. Each product was examined visually for clarity and the result in each case is indicated at the foot of the Table.

**TABLE**

| Ingredient | | Example | | | | | | | | | |
|---|---|---|---|---|---|---|---|---|---|---|---|
| | | 1 | 2 | 3 | 4 | 5 | 6 | 7 | 8 | 9 | 10 |
| Arquad 16/50 (50%) chloride | Cetyltrimethylammonium | 1.50 | | | | | | | | | |
| Crodafos N3A (100%) or Briphos 03D | Mono/di-oleyl ethoxy phosphate 3EO | - | - | - | - | - | - | - | - | 1.50 | 3.00 |
| Crodafos N5A (100%) | Mono/di-oleyl ethoxy phosphate, 5EO | 1.75 | - | - | - | - | - | - | - | - | - |
| Crodafos N10A (100%) | Mono/di-oleyl ethoxy phosphate, 10EO | - | 5.00 | - | - | - | - | - | - | - | - |
| Crodafos CS10 (100%) | Mono/di-cetostearyl ethoxy phosphate, 10EO | - | - | 5.00 | - | - | - | - | - | - | - |
| Crodafos G26A (100%) | Polyol ether phosphate ester, 26EO (mixture of mono/di esters) | - | - | - | 5.00 | - | - | - | - | - | - |
| Marlinat CM 40 (88-90%) C12-C14 fatty alcohol polyglycol ether carboxylic acid, 4EO | | - | - | - | - | 1.10 | - | - | - | - | - |
| Marlinat CM100 (85-90%) C12-C14 fatty alcohol polyglycol ether carboxylic acid, 10EO | | - | - | - | - | - | 1.70 | - | - | - | - |
| Akypo RS20 polyglycol ether carboxylic acid, 2EO | C18 fatty alcohol | - | - | - | - | - | - | 1.00 | - | - | - |
| Akypo RS60 polyglycol ether carboxylic acid, 6EO | C18 fatty alcohol | - | - | - | - | - | - | - | 1.40 | - | - |
| Natrosol 250 HHR | (Hydroxyethylcellulose) | 1.00 | | | | | | | | | |
| 1)Triton X-100 | (iso-octylphenoxypolyethoxyethanol) | 0.40 | | | | | | | | | |
| Perfume (OP 5368) | | 0.20 | | | | | | | | | |
| Sodium hydroxide | | to pH 4.5-5.5 | | | | | | | | | |
| Demineralised water | | to 100.00 | | | | | | | | | |
| CTAC:Anionic surfactant molar ratio | | 1:1 | 1:2 | 1:2 | 1:1 | 1:1 | 1:1 | 1:1 | 1:1 | 1:1 | 1:2 |
| Product appearance | | cloudy | clear | clear | clear | cloudy | clear | cloudy | cloudy | cloudy | cloudy |

1) non-ionic surfactant

It can be seen in the above Examples that the only hair conditioning compositions containing an anionic surfactant component in combination with a cationic surfactant component which are optically clear are those in which the anionic surfactant contains at least about 5 or 6 ethylene oxide residues per molecule.

TEST EXAMPLES

A series of comparative tests were conducted to demonstrate the hair conditioning benefits imparted by clear conditioning compositions in accordance with the invention. Amount of conditioning was as measured by Total Combing Time.

These comparative tests illustrate the advantageous properties of compositions within the scope of the present invention, and in particular demonstrate the importance of the cationic-anionic surfactant complex formed between the components being soluble, thereby giving a clear product, and also the optimisation of anionic to cationic surfactant ratio.

Method

Hair switches (10 g/10 in.) were prepared from Yugoslavian dark brown hair. Each switch was washed with 2 x 0.5g of 16% SLES.2EO and the time taken to untangle the hair was measured. The reading gives the base measurement for that switch. (The switch is combed starting at the bottom, easing the tangles out and moving in small steps up to the top of the switch. The Total Combing Time (TCT) is recorded by means of an electronic timer automatically actuated by the comb.) The switch was then washed with 2 x 0.5g of a non-conditioning shampoo and excess water removed by sliding the fingers down the full length of the switch. 1.0 g of test conditioner was applied 1 cm from the top of the switch and spread down the switch with a squeezing action. The product was left in contact with the hair for a total time of 1 minute before rinsing. Excess water was removed prior to measuring the time taken to untangle the hair after product application. This was compared against the base reading:

$$\% \, TCT \; = \; \frac{Product \, TCT}{Base \, TCT} \, x \, 100$$

Thus, the lower the figure for % TCT, the higher the conditioning benefit of the test product.

RESULTS

Test 1: The active ingredients in the products tested were as follows:

A - 1.5% Arquad 16/50
B - 5.0% Crodafos N10A
C - 1.5% Arquad 16/50 + 5.0% Crodafos N10A (Molar Ratio 1:2)

| PRODUCT | % TCT (mean +/- s.d., n = 3) |
|---------|------------------------------|
| A | 49.7 +/- 1.8 |
| B | 55.0 +/- 2.4 |
| C | 22.9 +/- 0.7 |

Test 2: The active ingredients in the products tested were as folows:

D - 1.5% Arquad 16/50 + 0.3% Crodafos N3A (Molar ratio 1:0.2)
E - 1.5% Arquad 16/50 + 0.5% Crodafos N10A (Molar ratio 1:0.2)
F - 1.5% Arquad 16/50 + 5.0% Crodafos N10A (Molar ratio 1:2)

| PRODUCT | % TCT (mean +/- s.d., n = 3) |
|---------|------------------------------|
| D | 52.6 +/- 2.4 |
| E | 36.8 +/- 2.2 |
| F | 21.4 +/- 0.6 |

Test 3: The active ingredients in the products tested were as follows:

G - 1.5% Arquad 16/50 + 0.5% Crodafos N10A (Molar ratio 1:0.2)
H - 1.5% Arquad 16/50 + 2.5% Crodafos N10A (Molar ratio 1:1)
I - 1.5% Arquad 16/50 + 5.0% Crodafos N10A (Molar ratio 1:2)

5

J - UK Cream Silk∗ Normal Conditioner

| PRODUCT | % TCT (mean +/- s.d., n = 3) |
|---------|------------------------------|
| G | 44.6 +/- 2.0 |
| H | 26.2 +/- 1.8 |
| I | 18.4 +/- 0.6 |
| J | 17.0 +/- 1.3 |

Test 4: The active ingredients in the products tested were as follows:

K - 1.5% Arquad 16/50 + 3.0% Briphos 03D (Molar ratio 1:2) (Cloudy)
L - 1.5% Arquad 16/50 + 5.0% Crodafos N10A (Molar ratio 1:2)
M - 1.5% Arquad 16/50 + 5.0% Crodafos N10A (Molar ratio 1:2)

| PRODUCT | % TCT (mean +/- s.d., n = 3) |
|---------|------------------------------|
| K | 40.7 +/- 0.5 |
| L | 20.5 +/- 0.6 |
| M | 18.4 +/- 1.3 |

**Claims**

1. A hair conditioning composition comprising:
   (a) at least one cationic surfactant component, and
   (b) at least one highly alkoxylated anionic surfactant component.

2. A hair conditioning composition according to claim 1, wherein the anionic surfactant component contains at least 5 alkylene oxide residues per molecule.

3. A hair conditioning composition according to claim 2, wherein the anionic surfactant contains from 5 or 6 to 30 ethylene oxide or propylene oxide residues per molecule.

4. A hair conditioning composition according to any preceding claim, wherein the highly alkoxylated anionic surfactant is selected from alkyl ether sulphates, alkyl ether phosphates and alkyl ether carboxylates each containing at least 5 alkylene oxide units per molecule.

5. A hair conditioning composition according to any preceding claim, wherein the cationic surfactant is a quaternary ammonium hydroxide or salt thereof.

6. A hair conditioning composition according to any preceding claim, wherein the molar ratio of component (b) to component (a) is at least 0.5:1.

7. A hair conditioning composition according to claim 6, wherein the said molar ratio is at least 1:1.

8. A hair conditioning composition according to any preceding claim, additionally comprising a thickening agent.

9. A hair conditioning composition according to any preceding claim, which is an aqueous composition comprising components (a) and (b) in a combined amount of from 0.1 to 90% by weight of the composition.

∗Trade Mark

10. A method of conditioning hair comprising applying thereto a composition according to any one of claims 1 to 9.